# EUROPEAN PATENT SPECIFICATION

(11) **EP 4 522 129 B1**
(45) Date of publication and mention of the grant of the patent: **28.10.2026**
(21) Application number: 23729579.5
(22) Date of filing: 10.05.2023
(51) Int. Cl.: A61K 9/14, A61K 9/48, A61K 31/437, A61P 3/04, A61P 3/10

(54) **GLP1 PHARMACEUTICAL COMPOSITIONS**
PHARMAZEUTISCHE GLP1-ZUSAMMENSETZUNGEN
COMPOSITIONS PHARMACEUTIQUES À BASE DE GLP1

(30) Priority: 11.05.2022 US 202263340591 P
(43) Date of publication of application: 19.03.2025
(73) Proprietor: ELI LILLY AND COMPANY, Indianapolis, IN 46285 (US)
(72) Inventor: BURNS, Lee Joseph, Indianapolis, Indiana 46206-6288 (US)
(74) Representative: Eli Lilly and Company Limited
(86) International application number: PCT/US2023/021637
(87) International publication number: WO 2023/220109

(56) References cited:
- WO-A2-02/056878
- WO-A2-02/064132
- WO-A2-2008/030524
- JP-A- 2019 099 571
- US-A1- 2019 225 604
- KAWAI TAKAHIRO ET AL: "Structural basis for GLP-1 receptor activation by LY3502970, an orally active nonpeptide agonist", PROCEEDINGS OF THE NATIONAL ACADEMY OF SCIENCES, vol. 117, no. 47, 24 November 2020 (2020-11-24), pages 29959 - 29967, XP093070947, ISSN: 0027-8424, Retrieved from the Internet <URL:http://dx.doi.org/10.1073/pnas.2014879117> DOI: 10.1073/pnas.2014879117

## Description

The present disclosure relates to oral capsule compositions of a GLP-1 receptor agonist, 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, (herein, GLP1RA), or a pharmaceutically acceptable salt thereof. Compositions, disclosed herein, can be useful for the treatment of type 2 diabetes mellitus (T2D) and in weight management.

Diabetes mellitus is a chronic disorder characterized by hyperglycemia resulting from defects in insulin secretion, insulin action, or both. In T2D, the combined effects of impaired insulin secretion and insulin resistance are associated with elevated blood glucose levels. T2D is an increasingly prevalent disease that frequently leads to declining health and quality of life for patients. Effective oral treatments to manage T2D and/or for use in weight management are desired.

GLP1RA, that is, 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof, is described and claimed in US10,858,356. The US10,858,356 patent generally describes oral compositions.

US2019225604 A1 and JP2019099571 A discloses certain GLP-1 receptor agonists and pharmaceutical compositions thereof.

GLP1RA may be prepared as a pharmaceutically acceptable salt. One salt of GLP1RA is a hemi-calcium hydrate, 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate, (herein "GLP1RA-Ca") with the structure as shown below.

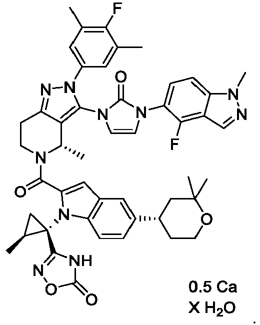

GLP1RA is a poorly permeable and poorly soluble weak acid with a pKa of 5.1. GLP1RA and its pharmaceutically acceptable salts have very low aqueous solubility across the physiologic pH range as well as in simulated physiological fluids. GLP1RA is observed to have a strong pH dependent solubility profile contributing to challenges such as variability in absorption and consequently in pharmacokinetic performance and potential food effects. There is a desire for GLP1RA, including but not limited to, GLP1RA-Ca, capsule compositions providing reliable PK performance in a patient friendly dosage form, with minimal potential for drug-drug interactions and reduced or no food effects. A GLP1RA composition to enhance solubility and dissolution rate of the active substance in a capsule dosage form may be desired. A pharmaceutically elegant dosage form to deliver an effective amount of active GLP1RA to the targeted portion of the gastrointestinal tract, while small enough to facilitate patient swallowing is desired.

Compositions described herein provide desired properties. In an aspect, the use of a spray dried dispersion (SDD) of the GLP1RA, or a pharmaceutically acceptable salt thereof, together with a pH modifier, as described herein, contributes to the desired properties. In an aspect, the specific particle sizes of the SDD and the particular compositions as described provide the desired properties. In an aspect, compositions disclosed herein provide desirable pharmacokinetic performances and deliver an effective amount of active GLP1RA to the targeted portion of the gastrointestinal tract. In an aspect, disclosed herein is an elegant dosage form that is convenient for patients to take without the limitation of food or water restrictions.

Solid oral formulations provided herein can be useful for patients in need of treatment for T2D. Solid oral formulations provided herein can be useful for patients in need of treatment for chronic weight management.

In an embodiment is a capsule composition comprising 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; and
a pH modifier.

In an embodiment is a composition wherein a pH modifier is selected from the group consisting of calcium carbonate, magnesium carbonate, sodium bicarbonate, sodium carbonate, magnesium hydroxide, calcium hydroxide, magnesium oxide, and a mixture thereof.

In an embodiment is a capsule composition comprising 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; and
a pH modifier; wherein the pH modifier is selected from the group consisting of calcium carbonate, anhydrous calcium carbonate, sodium bicarbonate, anhydrous sodium bicarbonate, sodium carbonate, anhydrous sodium carbonate, magnesium hydroxide, and anhydrous magnesium hydroxide.

In an embodiment, a pH modifier is sodium bicarbonate. In an embodiment, a pH modifier is sodium carbonate.

In an embodiment, a pH modifier is anhydrous. In an embodiment, a pH modifier is anhydrous sodium bicarbonate. In an embodiment, a pH modifier is anhydrous sodium carbonate.

In an embodiment is a capsule composition comprising 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; and
a pH modifier, wherein the pH modifier is anhydrous sodium bicarbonate.

In an embodiment is a composition wherein the composition comprises 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; and
a pH modifier, wherein the pH modifier is selected from sodium bicarbonate, anhydrous sodium bicarbonate, sodium bicarbonate hydrate, sodium carbonate, and anhydrous sodium carbonate.

In an embodiment is a composition wherein the composition comprises 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; and
a pH modifier, wherein the pH modifier is sodium bicarbonate.

In an embodiment is a composition wherein the composition comprises 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; and
a pH modifier, wherein the pH modifier is sodium bicarbonate.

In an embodiment is a composition wherein the composition comprises 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; and
a pH modifier, wherein the pH modifier is sodium carbonate.

In an embodiment, a capsule composition comprises 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one; and a pH modifier.

In an embodiment is a capsule composition comprising 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in the amount of about 0.5 to about 46 mg per capsule composition.

In an embodiment is a composition comprising 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in the amount of about 46 mg per capsule composition.

In an embodiment is a process for preparing a capsule composition as disclosed herein comprising an amorphous dispersion process. In an embodiment is a process for preparing a capsule composition as claimed herein comprising a spray dried dispersion (SDD) process.

In an embodiment, GLP1RA, or a pharmaceutically acceptable salt thereof, is prepared into a spray dried dispersion (SDD) for use as the active drug in a capsule composition. In an embodiment, an SDD of GLP1RA, or a pharmaceutically acceptable salt thereof, is prepared under the conditions as described in Example 2 or Alternative Example 2.

In an embodiment, the GLP1RA (3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one) or GLP1RA-Ca ( 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate) is present as an SDD preparation in a composition described herein.

In general, an SDD preparation disclosed herein comprises GLP1RA, or a pharmaceutically acceptable salt thereof, and a polymer to maintain an amorphous state of the GLP1RA, or a pharmaceutically acceptable salt thereof. In an embodiment, the polymer is selected from the group consisting of polyvinyl pyrrolidone (also known as "povidone" or "PVP") and polyvinyl pyrrolidone vinyl acetate (also known as "copovidone" or "PVP-VA"). In an embodiment, the polymer is PVP-VA. In an embodiment, the polymer is PVP.

In an embodiment where the weight percentage of the GLP1RA, or a pharmaceutically acceptable salt thereof, in an SDD preparation is specified, the balance component of the SDD is a polymer selected from PVP and PVP-VA, that is, the total weight percentage of the GLP1RA, or a pharmaceutically acceptable salt thereof, and the polymer is 100 wt%. In an embodiment, the polymer is PVP-VA. In an embodiment, the polymer is PVP. In an embodiment, a small or trace amount of the processing solvent or solvents may be present in an SDD preparation.

In an embodiment, the SDD preparation comprises about 20 wt% to about 40 wt% of GLP1RA or GLP1RA-Ca and the balance is composed of PVP-VA. In an embodiment, the SDD preparation comprises about 30 wt% of GLP1RA or GLP1RA-Ca and the balance is composed of PVP-VA.

In an embodiment, the mean particle size of the GLP1RA or GLP1RA-Ca SDD is about 5 µm to about 150 µm in diameter. In an embodiment, the mean particle size of the SDD is about 5 µm to about 113 µm in diameter. In an embodiment, the mean particle size of the SDD is about 40 µm to about 65 µm in diameter. In an embodiment, the mean particle size of the SDD is about 40 µm to about 50 µm in diameter. In an embodiment, the mean particle size of the SDD is about 5 µm to about 25 µm in diameter.

In an embodiment, the SDD preparation comprises about 20 wt% to about 40 wt% of GLP1RA-Ca and the balance is composed of PVP-VA. In an embodiment, the SDD preparation comprises about 30 wt% of GLP1RA or GLP1RA-Ca and the balance is composed of PVP-VA.

In an embodiment, the mean particle size of the GLP1RA-Ca SDD is about 5 µm to about 150 µm in diameter. In an embodiment, the mean particle size of the SDD is about 5 µm to about 113 µm in diameter. In an embodiment, the mean particle size of the SDD is about 40 µm to about 65 µm in diameter. In an embodiment, the mean particle size of the SDD is about 40 µm to about 50 µm in diameter. In an embodiment, the mean particle size of the SDD is about 5 µm to about 25 µm in diameter.

In an embodiment is a capsule composition comprising:
GLP1RA, or a pharmaceutically acceptable salt thereof;
a pH modifier selected from the group consisting of calcium carbonate, sodium bicarbonate, sodium carbonate, sodium carbonate hydrate, magnesium hydroxide, and a mixture thereof;
optionally, a filler selected from the group consisting of a sugar alcohol, microcrystalline cellulose, methylcellulose, hydroxypropylcellulose, hydroxypropyl methylcellulose, starch, dicalcium phosphate, and a mixture thereof; and
optionally, a glidant selected from the group consisting of colloidal silicon dioxide, talc, magnesium carbonate, and a mixture thereof.

In an embodiment is a composition comprising:
GLP1RA, or a pharmaceutically acceptable salt thereof; in an amount of about 0.7 mg to about 80 mg;
a pH modifier selected from the group consisting of calcium carbonate, sodium bicarbonate, sodium carbonate, sodium carbonate hydrate, magnesium hydroxide , and a mixture thereof; in an amount of about 20 mg to about 800 mg;
a filler selected from the group consisting of a sugar alcohol, microcrystalline cellulose, methylcellulose, hydroxypropylcellulose, hydroxypropyl methylcellulose, starch, dicalcium phosphate, and a mixture thereof; in an amount of about 0.1 mg to about 400 mg; and
optionally, a glidant selected from the group consisting of silicon oil, silicon dioxide, talc, magnesium carbonate, and a mixture thereof; in an amount of about 0 mg to about 20 mg.

In an embodiment is a composition comprising:
GLP1RA, or a pharmaceutically acceptable salt thereof; in an amount of about 0.7 mg to about 50 mg;
a pH modifier selected from the group consisting of sodium bicarbonate and sodium carbonate; in an amount of about 150 mg to about 700 mg; and
a glidant selected from the group consisting of silicon oil, silicon dioxide, talc, magnesium carbonate, and a mixture thereof; in an amount of about 0.1 mg to about 10 mg.

In an embodiment is a composition comprising:
GLP1RA, or a pharmaceutically acceptable salt thereof; in an amount of about 1 mg to about 45 mg;
a pH modifier which is sodium bicarbonate; in an amount of about 150 mg to about 650 mg;
a filler selected from the group consisting of a sugar alcohol, microcrystalline cellulose, methylcellulose, hydroxypropylcellulose, hydroxypropyl methylcellulose, starch, dicalcium phosphate, and a mixture thereof; in an amount of about 1 mg to about 150 mg; and
a glidant selected from the group consisting of silicon oil, silicon dioxide, talc, magnesium carbonate, and a mixture thereof; in an amount of about 0.1 mg to about 5 mg.

In an embodiment is a composition as described above, wherein the 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one ("GLP1RA"), or a pharmaceutically acceptable salt thereof, is in the form of an SDD preparation.

In an embodiment is a capsule composition comprising:
an SDD of about 20 wt% to about 40 wt% of GLP1RA, or a pharmaceutically acceptable salt thereof; and the balance of the SDD is composed of a polymer selected from PVP-VA and PVP; and
a pH modifier; wherein the pH modifier is selected from the group consisting of calcium carbonate, sodium bicarbonate, sodium carbonate, magnesium hydroxide, and anhydrous magnesium hydroxide.

In an embodiment is a composition as described above, wherein the SDD comprises about 30 wt% to about 35 wt% of GLP1RA, or a pharmaceutically acceptable salt thereof; and the balance of the SDD is composed of PVP-VA; and
a pH modifier; wherein the pH modifier is selected from the group consisting of sodium bicarbonate and sodium carbonate.

In an embodiment is a composition as described above, wherein the SDD comprises about 30 wt% of GLP1RA, or a pharmaceutically acceptable salt thereof; and the balance is composed of PVP-VA; wherein the SDD has a mean particle size of about 5 µm to about 113 µm in diameter; and a pH modifier which is sodium bicarbonate.

In an embodiment is a composition as described above, further comprising:
a filler selected from the group consisting of a sugar alcohol, microcrystalline cellulose, methylcellulose, hydroxypropylcellulose, hydroxypropyl methylcellulose, starch, dicalcium phosphate, and a mixture thereof; and
optionally, a glidant selected from the group consisting of silicon oil, silicon dioxide, talc, magnesium carbonate, and a mixture thereof.

In an embodiment is a capsule composition comprising:
GLP1RA, or a pharmaceutically acceptable salt thereof; in an amount of about 0.7 mg to about 50 mg;
a pH modifier which is sodium bicarbonate; in an amount of about 150 mg to about 650 mg;
a filler selected from the group consisting of a sugar alcohol, microcrystalline cellulose, methylcellulose, hydroxypropylcellulose, hydroxypropyl methylcellulose, starch, dicalcium phosphate, and a mixture thereof; in an amount of about 0.1 mg to about 80 mg; and
optionally, a glidant selected from the group consisting of silicon oil, silicon dioxide, talc, magnesium carbonate, and a mixture thereof; in an amount of about 0 mg to about 5 mg.

In an embodiment is a composition comprising:
an SDD of about 20 wt% to about 40 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; and the balance of the SDD is composed of a polymer selected from PVP-VA and PVP; and
a pH modifier; wherein the pH modifier is selected from the group consisting of calcium carbonate, sodium bicarbonate, sodium carbonate, magnesium hydroxide, anhydrous magnesium hydroxide, and a mixture thereof.

In an embodiment of the composition described above:
the SDD comprises about 30 wt% to about 35 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; and the balance of the SDD is composed of a polymer which is PVP-VA; and
a pH modifier; wherein the pH modifier is selected from the group consisting of sodium bicarbonate and sodium carbonate.

In an embodiment of the composition described above:
the SDD comprises about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; and the balance of the SDD is composed of PVP-VA; wherein the SDD has a mean particle size of about 5 µm to about 113 µm in diameter; and
a pH modifier which is sodium bicarbonate.

In an embodiment of the compositions described above, the compositions further comprise:
optionally, a filler selected from the group consisting of a sugar alcohol, microcrystalline cellulose, methylcellulose, hydroxypropylcellulose, hydroxypropyl methylcellulose, starch, dicalcium phosphate, and a mixture thereof; and
optionally, a glidant selected from the group consisting of silicon oil, silicon dioxide, talc, magnesium carbonate, and a mixture thereof.

In an embodiment, a capsule composition comprises:
3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; in an amount of about 0.7 mg to about 50 mg, on a free acid basis;
a pH modifier which is sodium bicarbonate; in an amount of about 150 mg to about 650 mg;
optionally, a filler selected from the group consisting of a sugar alcohol, microcrystalline cellulose, methylcellulose, hydroxypropylcellulose, hydroxypropyl methylcellulose, starch, dicalcium phosphate, and a mixture thereof; in an amount of about 0 mg to about 200 mg; and
optionally, a glidant selected from the group consisting of silicon oil, silicon dioxide, talc, magnesium carbonate, and a mixture thereof; in an amount of about 0 mg to about 5 mg.

In an embodiment of the composition described above, the composition comprises:
an SDD of about 30 wt% to about 35 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 0.7 mg to about 45 mg, on a free acid basis; and the balance of the SDD is composed of PVP-VA;
a pH modifier which is sodium bicarbonate; in an amount of about 200 mg to about 650 mg;
optionally, a filler selected from the group consisting of a sugar alcohol, microcrystalline cellulose, methylcellulose, hydroxypropylcellulose, hydroxypropyl methylcellulose, starch, dicalcium phosphate, and a mixture thereof; in an amount of about 0 mg to about 60 mg; and
optionally, a glidant selected from the group consisting of silicon oil, silicon dioxide, talc, magnesium carbonate, and a mixture thereof; in an amount of about 0 mg to about 5 mg.

In an embodiment of the composition described above, the composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of between about 1 mg to about 36 mg, on a free acid basis; and the balance of the SDD is composed of PVP-VA;
a pH modifier which is sodium bicarbonate; in an amount of about 200 mg to about 600 mg;
optionally, a filler selected from the group consisting of a sugar alcohol, microcrystalline cellulose, methylcellulose, hydroxypropylcellulose, hydroxypropyl methylcellulose, starch, dicalcium phosphate, and a mixture thereof; in an amount of about 0 mg to about 60 mg; and
optionally, a glidant selected from the group consisting of silicon oil, silicon dioxide, talc, magnesium carbonate, and a mixture thereof; in an amount of about 0 mg to about 5 mg.

In an embodiment of the compositions described above, the SDD has a mean particle size of about 5 µm to about 113 µm in diameter.

In an embodiment of the composition described above:
the filler, when present, is MCC PH-102; in an amount of about 2 mg to about 25 mg; and
the glidant, when present, is silicon oil or silicon dioxide; in an amount of about 0.1 mg to about 5 mg.

In an embodiment, a composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 1 mg, on a free acid basis;
a pH modifier which is sodium bicarbonate; in an amount of about 200 mg;
a filler which is MCC PH-102; in an amount of about 40 mg to about 50 mg; and
a glidant which is silicon oil or silicon dioxide; in an amount of about 1 mg to about 5 mg;
wherein the total weight of the composition is about 250 mg.

In an embodiment, a composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 1 mg (on a free acid basis);
a pH modifier which is sodium bicarbonate; in an amount of about 200 mg;
a filler which is MCC PH-102; in an amount of about 44 mg; and
a glidant which is silicon oil or silicon dioxide; in an amount of about 2.5 mg;
wherein the total weight of the composition is about 250 mg.

In an embodiment, a composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 16 mg, on a free acid basis;
a pH modifier which is sodium bicarbonate; in an amount of about 200 mg;
a filler which is MCC PH-102; in an amount of about 5 mg to about 10 mg; and
a glidant which is silicon oil or silicon dioxide; in an amount of about 1 mg to about 5 mg;
wherein the total weight of the composition is about 260 mg.

In an embodiment, a composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 16 mg (on a free acid basis);
a pH modifier which is sodium bicarbonate; in an amount of about 200 mg;
a filler which is MCC PH-102; in an amount of about 7 mg; and
a glidant which is silicon oil or silicon dioxide; in an amount of about 2.6 mg;
wherein the total weight of the composition is about 260 mg.

In an embodiment, a composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 6 mg, on a free acid basis; and about 70 wt% of PVP-VA in an amount of about 14 mg;
a pH modifier which is sodium bicarbonate; in an amount of about 600 mg; and
a glidant which is silicon oil; in an amount of about 1 mg to about 10 mg.

In an embodiment, a composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 6 mg (on a free acid basis); and about 70 wt% of PVP-VA in an amount of about 14 mg;
a pH modifier which is sodium bicarbonate; in an amount of about 600 mg; and
a glidant which is silicon oil or silicon dioxide; in an amount of about 6 mg.

In an embodiment, a composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 12 mg, on a free acid basis; and about 70 wt% of PVP-VA in an amount of about 28 mg;
a pH modifier which is sodium bicarbonate; in an amount of about 600 mg; and
a glidant which is silicon oil; in an amount of about 1 mg to about 10 mg.

In an embodiment, a composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 12 mg (on a free acid basis); and about 70 wt% of PVP-VA in an amount of about 28 mg;
a pH modifier which is sodium bicarbonate; in an amount of about 600 mg; and
a glidant which is silicon oil or silicon dioxide; in an amount of about 6 mg.

In an embodiment, a composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 36 mg, on a free acid basis; and about 70 wt% of PVP-VA in an amount of about 83 mg;
a pH modifier which is sodium bicarbonate; in an amount of about 600 mg; and
a glidant which is silicon oil; in an amount of about 1 mg to about 10 mg.

In an embodiment, a composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 36 mg, on a free acid basis; and about 70 wt% of PVP-VA in an amount of about 83 mg;
a pH modifier which is sodium bicarbonate; in an amount of about 600 mg; and
a glidant which is silicon oil or silicon dioxide; in an amount of about 6 mg.

In an embodiment, the compositions described above are encapsulated in a capsule shell. In an embodiment, the capsule shell is an HPMC capsule shell.

Certain abbreviations are defined as follows: "cfm" refers to cubic feet/minute; "Cmax" refers to the maximum plasma concentration that a drug achieves in tested area after the drug has been administered and prior to the administration of a second dose; "DDI" refers to drug-drug interactions; "DR" refers to delayed release; "EtOH" refers to ethanol or ethyl alcohol; "FaSSiF" refers to fasted state simulated intestinal fluid; "FaSSGF" refers to fasted state simulated gastric fluid; "FeSSIF" refers to fed state simulated intestinal fluid; "hr" refers to hour; "hrs" refers to hours; "IR" refers to immediate release; "PK" refers to pharmacokinetics; "MeOH" refers to methanol or methyl alcohol; "rpm" refers to revolutions per minute; "PVP-VA" refers to polyvinylpyrolido/vinyl acetate copolymer; "SDD" refers to spray dried dispersion "SIF" refers to simulated intestinal fluid; "T2D" refers to type 2 diabetes; "THF" refers to tetrahydrofuran; "USP" refers to United States Pharmacopeia; "wt%" refers to mass of desired material/total mass; and XRPD refers to X-ray powder diffraction. As used herein "prep" means to preparation.

A skilled artisan may prepare 3-[(1S,2S)-1-[5-[(4S)-2,2-Dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca, spray dried dispersion (SDD) preparation under a variety of processing conditions and with varying equipment to produce material of suitable quality and critical characteristics. In an embodiment, product is amorphous as measured by XRPD, of the desired wt% of drug (30%) and PVP-VA (70%), of a particle size that can be isolated and forward processed and is acceptably free of process related impurities and excessive residual solvents. In an embodiment, an amorphous solid dispersion may be used in the preparation process. In an embodiment, the particle size is from about 5 to about 113 µm for use in preparing the composition for use in the preparing the composition.

### Example 1

### 3-[(1S,2S)-1-[5-[(4S)-2,2-Dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate

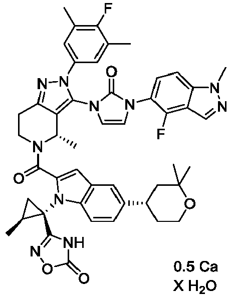

Example 1 may be prepared as described in WO 2018/056453.

The title compound has alternative names. For example, it is also known as the hemicalcium salt hydrate of orforglipron.

Another name for the title compound is 1,2,4-oxadiazol-5(2H)-one, 3-[(1S,2S)-1-[2-[[(4S)-2-(4-fluoro-3,5-dimethylphenyl)-3-[3-(4-fluoro-1-methyl-1H-indazol-5-yl)-2,3-dihydro-2-oxo-1H-imidazol-1-yl]-2,4,6,7-tetrahydro-4-methyl-5H-pyrazolo[4,3-c]pyridin-5-yl]carbonyl]-5-[(4S)-tetrahydro-2,2-dimethyl-2H-pyran-4-yl]-1H-indol-1-yl]-2-methylcyclopropyl]-, calcium salt (2:1), hydrate.

### Example 2

### 3-[(1S,2S)-1-[5-[(4S)-2,2-Dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate, SDD preparation

30% 3-[(1S,2S)-1-[5-[(4S)-2,2-Dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate SDD is prepared by dissolving 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate (8.7 g, 92% potency) and polyvinylpyrolido/vinyl acetate copolymer (PVP-VA, Kollidon^{®} VA64) (18.7 g) in EtOH (200 mL) at room temperature. Following dissolution of the solids, the solution is spray dried on a conventional spray dryer with a pressure nozzle. The following parameters in Table 1 are used to create the dispersion with a Buchi B290/B295. Spray drying can begin when the spray dryer temperature is above 33 °C. Collect the material and dry under vacuum at 50 °C overnight to give the title compound (21.99 g, 7.0 g, 92% potency, 80% recovery) and observe via photomicroscopy that the material is microscopically non birefringent particles that are approximately 5-25 µm in diameter.

### SDD Parameters

**Table 1**

| Process Parameter | Value |
|---|---|
| Aspirator | 100% |
| Nebulization Flow | 37 mm (~400 L/hr) |
| Solution flow rate | 40% (12.5 mL/min) |
| Chiller Temperature | -20 °C |
| Drying Gas Inlet Temperature | 120 °C |
| Spray Dryer Outlet Temperature (°C) | 75 °C |

### Alternate Example 2

### 3-[(1S,2S)-1-[5-[(4S)-2,2-Dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate, SDD preparation

3-[(1S,2S)-1-[5-[(4S)-2,2-Dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate, is dissolved in ethanol, denatured with methanol (5% v/v or w/w). A 20% w/w solids solution is prepared with 30% w/w of the solid fraction composed of the title compound (on a free acid basis) and the balance is composed of PVP-VA. This translates to 6% of the title compound (on a free acid basis), 14% PVP-VA and 80% of denatured ethanol SDA-3A - all fractions as w/w. After spray drying, the solids that form are composed of 30% w/w of the solid fraction composed of the title compound (on a free acid basis) and the balance is composed of PVP-VA. The % values are shown in the Table 2 below.

### Formulation of Alternate Example 2 SDD

**Table 2**

| | Solution (mass fractions) | Amorphous SDD (mass fraction) |
|---|---|---|
| Title compound | 6% | 30.0% |
| PVP-VA | 14% | 70.0% |
| EtOH SDA-3A | 80% | |
| EtOH | *76%* | |
| MeOH | *4%* | |

Once a solution is prepared, the solution is pumped to a spray dryer where the solution atomizes upon entry. Heated drying gas enters co-current to the atomized liquid at the top of the spray drying chamber at an approximate ratio of 0.044 kg/kg of spray solution to drying gas. The inlet temperature is adjusted to provide an outlet temperature of 35 to 45 °C. The solids formed in the spray dryer are collected from a cyclone as well as a filter housing on the gas stream. The gas passed over a condenser maintained at -3 °C, to remove (to a dewpoint of -3 °C) solvent. The gas is then heated to the inlet temperature and passed back to the spray dryer.

### Example 3

### Capsule Formulation

### 3-[(1S,2S)-1-[5-[(4S)-2,2-Dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate, 1 mg and 15 mg capsule formulation

Dispense the SDD, NaHCO₃, microcrystalline cellulose (MCC PH-102), and SiO₂ into separate low-density polyethylene (LDPE) bags. Pass NaHCO₃ and MCC PH-102 through a screen (30 mesh) sequentially into separate LDPE bags. Add ~25% of the sieved NaHCO₃ to a 10 L mixing bin. Add sieve MCC to the bag containing the SDD and manually mix for at least two minutes. Then add SiO₂ to the MCC and SDD blend and manually mix for at least two more minutes. Sieve the blend through a 30 mesh screen into the bin. Rinse the blend LDPE bag with the remaining NaHCO₃ and sieve through a 30 mesh screen into the bin. Blend for 15 minutes at 20 RPM. Sieve the blend through a 30 mesh screen and blend again for 15 min at 20 RPM. Fill the blend into empty 0 capsule shells to the target fill weight using a semi-automated filling machine (e.g. Dott-Bonapace).

| | Function | Ex 3 capsule, 1 mg, mg/capsule | Ex 3 capsule, 15 mg, mg/capsule |
|---|---|---|---|
| Alt. Ex 2 SDD | | 3.3* | 50* |
| NaHCO₃ | pH modifier | 200 | 200 |
| MCC PH-102 | filler/binder | 44.1 | 7.1 |
| SiO₂, SYLOID 244 FP (1%) | glidant | 2.5 | 2.6 |
| Total (mg) | | 250.0 | 260.0 |

| | | | |
|---|---|---|---|
| * As described herein, the conversion factor for GLP1RA/GLP1RA-Ca (i.e., the hemicalcium salt hydrate of GLP1RA) is assumed to be around 0.91. As a skilled artisan readily appreciates, the exact conversion rate may change slightly depending on the actual content of the hydrate. | | | |

### Example 4

### Capsule Preparation

### 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate (13.3 % w/w)

### Capsule Preparation

Capsules are prepared by first adding sodium bicarbonate (600 mg) to a size 0 hypromellose, short for hydroxypropyl methylcellulose (HPMC), capsule followed by 10 mg of SDD to yield capsules of 3 mg strength of the active moiety.

### In Vitro Dissolution Test of Capsules

In vitro dissolution of capsules is conducted using USP apparatus II with the following conditions: paddle speed of 50 rpm until 60 minutes, and 250 rpm from 60 to 90 minutes, 900 mL of 0.01N hydrochloric acid held at 37 °C, and the use of basket sinkers to mitigate capsule buoyancy. 6 replicates are tested, and samples are taken from the dissolution vessels at 10, 15, 20, 30, 45, 60 and 90 minutes and the amount of drug dissolved against label claim is detected using conventional HPLC with UV detection. The results can be found in the table below.

| | **Percent (%) of drug dissolved at Time (min)** | | | | | | |
|---|---|---|---|---|---|---|---|
| **Vessel** | **10** | **15** | **20** | **30** | **45** | **60** | **90** |
| **1.00** | 0.00 | 0.99 | 2.22 | 4.15 | 6.72 | 8.62 | 18.79 |
| **2.00** | 1.01 | 3.28 | 4.28 | 6.38 | 6.89 | 7.01 | 11.50 |
| **3.00** | 0.00 | 0.89 | 1.82 | 4.39 | 10.17 | 12.78 | 17.01 |
| **4.00** | 0.00 | 1.35 | 1.64 | 6.59 | 9.40 | 12.61 | 14.51 |
| **5.00** | 0.00 | 0.60 | 1.30 | 3.58 | 6.12 | 7.12 | 9.96 |
| **6.00** | 0.00 | 0.80 | 2.50 | 3.45 | 7.26 | 10.64 | 14.17 |
| **Ave** | 0.17 | 1.32 | 2.29 | 4.76 | 7.76 | 9.80 | 14.32 |
| **StDev** | 0.41 | 0.99 | 1.06 | 1.39 | 1.63 | 2.60 | 3.29 |
| **RSD** | 244.95 | 75.28 | 46.25 | 29.15 | 20.99 | 26.57 | 22.99 |

### Clinical Study Results

Surprisingly despite relatively poor dissolution in 0.01N HCl, when tested in clinical study GZGA, the capsule prepared with SDD and sodium bicarbonate showed an increase in both Cmax and AUC as compared to a capsule with only SDD.

| Capsule Formulation | No. of healthy subjects who received dose | Cmax (ng/mL) | AUC_{0_∞} (ng*h/mL) |
|---|---|---|---|
| 3.3 mg GLP1RA-Ca (as 10 mg SDD) + 600 mg sodium bicarbonate capsule | 4 | 14.9 | 387 |
| 3.3 mg GLP1RA-Ca (as 10 mg SDD) Capsule | 6 | 8.97 | 229 |

### Example 4

### Capsule Preparation

### 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate

### GLP-1RA Unit Formulas (1-8 mg)

| Component | Quantity (mg/capsule) | | | | | | Function |
|---|---|---|---|---|---|---|---|
| | 1 mg | 2 mg | 3 mg | 4 mg | 6 mg | 8 mg | |
| Spray Dried Dispersion Components | | | | | | | |
| GLP1RA Hemicalcium ^{A} | 1.0 | 2.0 | 3.1 | 4.1 | 6.1 | 8.2 | Active Ingredient |
| *GLP1RA- Free Acid* | *1.0* | *2.0* | *3.0* | *4. 0* | *6.0* | *8.0* | |
| Polyvinyl Pyrrolidone Vinyl Acetate | 2.3 | 4.6 | 6.9 | 9.2 | 13.9 | 18.5 | Dispersion Polymer |
| Ethanol | --^{B} | --^{B} | --^{B} | --^{B} | --^{B} | --^{B} | Solvent |
| Methanol | --^{B} | --^{B} | --^{B} | --^{B} | --^{B} | --^{B} | Solvent |

| Encapsulation Components | | | | | | | |
|---|---|---|---|---|---|---|---|
| GLP1RA-0.5Ca SDD^{C} | 3.3 | 6.7 | 10.0 | 13.3 | 20.0 | 26.7 | Active Dispersion |
| Sodium Bicarbonate | 600.0 | 600.0 | 600.0 | 600.0 | 600.0 | 600.0 | pH modifier |
| Silicone Oil | 6.061 | 6.061 | 6.061 | 6.061 | 6.061 | 6.061 | Lubricant |
| HPMC Capsule Shell (pieces) | 1 | 1 | 1 | 1 | 1 | 1 | Capsule shell |
| Total Capsule Fill Weight: | 609.4 | 612.8 | 616.1 | 619.4 | 626.1 | 632.8 | |

| | | | | | | | |
|---|---|---|---|---|---|---|---|
| A The quantity of GLP1RA-0.5Ca hydrate may be adjusted based on the release potency to maintain target concentration in the SDD. The conversion factor of GLP1RA/GLP1RA hemicalcium is about 0.98. B Ethanol and methanol are removed to residual levels during drying. C If necessary, the amount of SDD may be adjusted to account for assay. The total capsule fill weight will be adjusted accordingly. | | | | | | | |

### GLP-1RA Capsule Unit Formulas (12 - 45 mg)

| Component | Quantity (mg/capsule) | | | | | Function |
|---|---|---|---|---|---|---|
| | 12 mg | 16 mg | 24 mg | 36 mg | 45 mg | |
| Spray Dried Dispersion Components | | | | | | |
| GLP1RA Hemicalcium ^{A} | 12.3 | 16.3 | 24.5 | 36.8 | 46.0 | Active Ingredient |
| *GLP1RA Free Acid* | *12.0* | *16.0* | *24.0* | *36.0* | *45.0* | |
| Polyvinyl Pyrrolidone Vinyl Acetate | 27.7 | 37.0 | 55.5 | 83.2 | 104.0 | Dispersion Polymer |
| Ethanol | --^{B} | --^{B} | --^{B} | --^{B} | --^{B} | Solvent |
| Methanol | --^{B} | --^{B} | --^{B} | --^{B} | --^{B} | Solvent |

| Encapsulation Components | | | | | | |
|---|---|---|---|---|---|---|
| GLP1RA-Ca:SDD^{C} | 40.0 | 53.3 | 80.0 | 120.0 | 150.0 | Active Dispersion |
| Sodium Bicarbonate | 600.0 | 600.0 | 600.0 | 600.0 | 600.0 | pH modifier |
| Silicone Oil | 6.0 | 6.0 | 6.0 | 6.0 | 6.0 | Lubricant |
| HPMC Capsule Shell (pieces) | 1 | 1 | 1 | 1 | 1 | Capsule shell |
| Total Capsule Fill Weight: | 646.1 | 659.4 | 686.1 | 726.1 | 756.1 | |

| | | | | | | |
|---|---|---|---|---|---|---|
| A The quantity of GLP1RA-0.5Ca hydrate may be adjusted based on the release potency to maintain target concentration in the SDD. The conversion factor of GLP1RA/GLP1RA hemicalcium is about 0.98. B Ethanol and methanol are removed to residual levels during drying. C If necessary, the amount of SDD may be adjusted to account for assay. The total capsule fill weight will be adjusted accordingly. | | | | | | |

### Example 5

### Particle Size Measurement

Particle size of the SDD was determined by laser diffraction utilizing wet dispersion on the Malvern Mastersizer 3000 equipped with the Hydro MV (medium volume) liquid disperser (Malvern Instruments Ltd. UK.). Optical model: Mie model, obscuration limits: 5-30%, general purpose model. The volume-based distribution was measured and the (D10, D50, D90) quantiles were reported.

The SDDs of Example 2 and Alternate Example 2 have a mean particle size of about 40 to about 65 µm, or more specifically, about 40 to about 50 µm, in diameter as measured by the above method.

### Manufacturing Process

The amorphous solid dispersion, consisting of 30 wt% GLP1RA free acid, is manufactured separately using a spray drying process. GLP1RA-Ca and PVP/VA are dissolved in a solvent mixture containing ethanol and methanol. The mixture is spray dried at an elevated temperature with a stream of nitrogen to remove solvent. The process renders the drug substance as an amorphous solid dispersion of GLP1RA-Ca in a matrix of PVP/VA. The GLP1RA-Ca SDD may be dried for further reduction of residual solvent levels.

For the encapsulation process, GLP1RA-Ca SDD is filled into capsules using automated equipment as the first fill in a two-fill process. Sodium bicarbonate with silicone 1% is filled into capsules using automated equipment as the second fill. Sodium bicarbonate with silicone 1% is manufactured separately using a liquid addition blending process such as high shear ring layer mixer, liquid capable tumble bin with I-bar, continuous twin screw wet granulation, or ribbon blending. The dual powder fill strategy addresses powder segregation that may occur with very low drug load, difference in particle size and density between the GLP1RA-Ca SDD and sodium bicarbonate. This encapsulation process allows for batches as small as 12 capsules, while accommodating substantially larger batch size, for example, but not limited to, over one hundred thousand capsules per batch.

As an alternative encapsulation process, GLP1RA-Ca SDD may be first blended with the mixture of sodium bicarbonate with silicone 1% and then filled into capsules using automated equipment.

## Claims

1. A solid oral capsule composition comprising a spray dried dispersion (SDD) of
3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; and
a pH modifier selected from the group consisting of calcium carbonate, magnesium carbonate, sodium bicarbonate, sodium carbonate, magnesium hydroxide, calcium hydroxide, magnesium oxide, and a mixture thereof.

2. The composition as claimed by Claim 1, wherein the pH modifier is selected from the group consisting of calcium carbonate, anhydrous calcium carbonate, sodium bicarbonate, anhydrous sodium bicarbonate, sodium carbonate, anhydrous sodium carbonate, magnesium hydroxide, and anhydrous magnesium hydroxide.

3. The composition as claimed by Claim 1 or Claim 2, wherein the pH modifier is sodium bicarbonate.

4. The composition as claimed by Claim 3, wherein the pH modifier is anhydrous sodium bicarbonate.

5. The composition as claimed by any one of Claims 1 to 4, wherein the composition comprises
3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one; and
anhydrous sodium bicarbonate.

6. The composition as claimed by any one of Claims 1 to 4, wherein the 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof, is
3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate.

7. The composition as claimed by claim 6, wherein the 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof, is
3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate in an amount of between about 1.2 to about 46 mg, on a free acid basis, per capsule composition.

8. The composition as claimed by any one of Claims 1 to 4, wherein the 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof, is in an amount of between about 1 mg to about 45 mg, on a free acid basis, per capsule.

9. A composition comprising:
a spray dried dispersion (SDD) of about 30 wt% to about 35 wt% of 3-[(1S,2S)-1-5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; in an amount of between about 0.7 mg to about 50 mg, on a free acid basis; and the balance of the SDD is composed of PVP-VA; and
a pH modifier selected from the group consisting of calcium carbonate, magnesium carbonate, sodium bicarbonate, sodium carbonate, magnesium hydroxide, calcium hydroxide, magnesium oxide, and a mixture thereof.

10. The composition as claimed by Claim 9, wherein the composition comprises:
the SDD of about 30 wt% 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, or a pharmaceutically acceptable salt thereof; in an amount of between about 0.7 mg to about 45 mg, on a free acid basis;
a pH modifier which is sodium bicarbonate; in an amount of about 150 mg to about 650 mg;
optionally, a filler selected from the group consisting of a sugar alcohol, microcrystalline cellulose, methylcellulose, hydroxypropylcellulose, hydroxypropyl methylcellulose, starch, dicalcium phosphate, and a mixture thereof; in an amount of about 0 mg to about 200 mg; and
optionally, a glidant selected from the group consisting of silicon oil, silicon dioxide, talc, magnesium carbonate, and a mixture thereof; in an amount of about 0 mg to about 5 mg.

11. The composition as claimed by Claim 10, wherein the composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of between about 1 mg to about 36 mg, on a free acid basis; and the balance of the SDD is composed of PVP-VA;
a pH modifier which is sodium bicarbonate; in an amount of about 200 mg to about 600 mg;
optionally, a filler selected from the group consisting of a sugar alcohol, microcrystalline cellulose, methylcellulose, hydroxypropylcellulose, hydroxypropyl methylcellulose, starch, dicalcium phosphate, and a mixture thereof; in an amount of about 0 mg to about 60 mg; and
optionally, a glidant selected from the group consisting of silicon oil, silicon dioxide, talc, magnesium carbonate, and a mixture thereof; in an amount of about 0 mg to about 5 mg.

12. The composition as claimed by Claim 11, wherein:
no filler is present; and
the glidant is silicon oil; in an amount of about 0.1 mg to about 5 mg.

13. The composition as claimed by any one of Claims9-12, wherein the SDD has a mean particle size of about 5 µm to about 113 µm in diameter.

14. The composition as claimed by Claim 11, wherein the composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 1 mg, on a free acid basis;
a pH modifier which is sodium bicarbonate; in an amount of about 200 mg;
a filler which is MCC PH-102; in an amount of about 40 mg to about 50 mg; and
a glidant which is silicon oil or silicon dioxide; in an amount of about 1 mg to about 5 mg;
wherein the total weight of the composition is about 250 mg.

15. The composition as claimed by Claim 11, wherein the composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 6 mg, on a free acid basis; and about 70 wt% of PVP-VA in an amount of about 14 mg;
a pH modifier which is sodium bicarbonate; in an amount of about 600 mg; and
a glidant which is silicon oil; in an amount of about 1 mg to about 10 mg.

16. The composition as claimed by Claim 11, wherein the composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 12 mg, on a free acid basis; and about 70 wt% of PVP-VA in an amount of about 28 mg;
a pH modifier which is sodium bicarbonate; in an amount of about 600 mg; and
a glidant which is silicon oil; in an amount of about 1 mg to about 10 mg.

17. The composition as claimed by Claim 11, wherein the composition comprises:
an SDD of about 30 wt% of 3-[(1S,2S)-1-[5-[(4S)-2,2-dimethyloxan-4-yl]-2-[(4S)-2-(4-fluoro-3,5-dimethylphenyl)-3-[3-(4-fluoro-1-methylindazol-5-yl)-2-oxoimidazol-1-yl]-4-methyl-6,7-dihydro-4H-pyrazolo[4,3-c]pyridine-5-carbonyl]indol-1-yl]-2-methylcyclopropyl]-4H-1,2,4-oxadiazol-5-one, 0.5 Ca hydrate; in an amount of about 36 mg, on a free acid basis; and about 70 wt% of PVP-VA in an amount of about 83 mg;
a pH modifier which is sodium bicarbonate; in an amount of about 600 mg; and
a glidant which is silicon oil; in an amount of about 1 mg to about 10 mg.

18. The composition as claimed by any one of Claims 14-17, wherein the SDD has a mean particle size of about 5 µm to about 113 µm in diameter.

19. The composition as claimed by Claim 18, wherein the SDD has a mean particle size of about 40 µm to about 65 µm in diameter.

20. The composition as claimed by any one of Claim 19, wherein the composition is in a capsule shell.

21. The composition as claimed by any one of Claims 20, wherein the capsule shell is an HPMC capsule shell.

22. The composition as claimed by any one of Claims 1 to 21 for use in therapy.

23. The composition for use according to claim 22, wherein the therapy is treating type 2 diabetes.

24. The composition for use according to claim 22, wherein the therapy is weight management.

## Patentansprüche

1. Feste Zusammensetzung für eine Kapsel zur oralen Einnahme, umfassend eine sprühgetrocknete Dispersion (SDD) von 3-[(1S,2S)-1-[5-[(4S)-2,2-Dimethyloxan-4-yl]-2-[(4S)-2-(4-fluor-3,5- dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on oder ein pharmazeutisch verträgliches Salz davon; und
einen pH-Modifikator, ausgewählt aus der Gruppe bestehend aus Calciumcarbonat, Magnesiumcarbonat, Natriumbicarbonat, Natriumcarbonat, Magnesiumhydroxid, Calciumhydroxid, Magnesiumoxid und einer Mischung davon.

2. Zusammensetzung nach Anspruch 1, wobei der pH-Modifikator ausgewählt ist aus der Gruppe bestehend aus Calciumcarbonat, wasserfreiem Calciumcarbonat, Natriumbicarbonat, wasserfreiem Natriumbicarbonat, Natriumcarbonat, wasserfreiem Natriumcarbonat, Magnesiumhydroxid und wasserfreiem Magnesiumhydroxid.

3. Zusammensetzung nach Anspruch 1 oder Anspruch 2, wobei der pH-Modifikator Natriumbicarbonat ist.

4. Zusammensetzung nach Anspruch 3, wobei der pH-Modifikator wasserfreies Natriumbicarbonat ist.

5. Zusammensetzung nach einem der Ansprüche 1 bis 4, wobei die Zusammensetzung umfasst: 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on; und
wasserfreies Natriumbicarbonat.

6. Zusammensetzung nach einem der Ansprüche 1 bis 4, wobei das 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, oder ein pharmazeutisch verträgliches Salz davon, Folgendes ist: 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, 0,5-Ca-Hydrat.

7. Zusammensetzung nach Anspruch 6, wobei das 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, oder ein pharmazeutisch verträgliches Salz davon, Folgendes ist: 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, 0,5-Ca-Hydrat in einer Menge zwischen etwa 1,2 bis etwa 46 mg, bezogen auf die freie Säure, pro Kapselzusammensetzung.

8. Zusammensetzung nach einem der Ansprüche 1 bis 4, wobei das 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H- pyrazolo[4,3-c]pyridin-5-carbonyl]indol-1-yl]-2-methylcyclopropyl]-4H-1,2,4-oxadiazol-5-on oder ein pharmazeutisch verträgliches Salz davon in einer Menge zwischen etwa 1 mg bis etwa 45 mg, bezogen auf die freie Säure, pro Kapsel vorliegt.

9. Zusammensetzung, umfassend:
eine sprühgetrocknete Dispersion (SDD) von etwa 30 Gew.-% bis etwa 35 Gew.-% von 3-[(1S,2S)-1-[5-[(4S)-2,2-dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, oder ein pharmazeutisch verträgliches Salz davon; in einer Menge zwischen etwa 0,7 mg bis etwa 50 mg, bezogen auf die freie Säure; und der Rest der SDD besteht aus PVP-VA; und
einen pH-Modifikator, ausgewählt aus der Gruppe bestehend aus Calciumcarbonat, Magnesiumcarbonat, Natriumbicarbonat, Natriumcarbonat, Magnesiumhydroxid, Calciumhydroxid, Magnesiumoxid und einer Mischung davon.

10. Zusammensetzung nach Anspruch 9, wobei die Zusammensetzung umfasst:
die SDD von etwa 30 Gew.-% 3-[(1S,2S)-1-[5-[(4S)-2,2-dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, oder ein pharmazeutisch verträgliches Salz davon; in einer Menge zwischen etwa 0,7 mg bis etwa 45 mg, bezogen auf die freie Säure;
einen pH-Modifikator, der Natriumbicarbonat ist; in einer Menge von etwa 150 mg bis etwa 650 mg;
gegebenenfalls einen Füllstoff, ausgewählt aus der Gruppe bestehend aus einem Zuckeralkohol, mikrokristalliner Cellulose, Methylcellulose, Hydroxypropylcellulose, Hydroxypropylmethylcellulose, Stärke, Dicalciumphosphat und einer Mischung davon; in einer Menge von etwa 0 mg bis etwa 200 mg; und
gegebenenfalls ein Fließregulierungsmittel, ausgewählt aus der Gruppe bestehend aus Silikonöl, Siliziumdioxid, Talkum, Magnesiumcarbonat und einer Mischung davon; in einer Menge von etwa 0 mg bis etwa 5 mg.

11. Zusammensetzung nach Anspruch 10, wobei die Zusammensetzung umfasst:
eine SDD von etwa 30 Gew.-% von 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, 0,5-Ca-Hydrat; in einer Menge zwischen etwa 1 mg bis etwa 36 mg, bezogen auf die freie Säure; und der Rest der SDD besteht aus PVP-VA;
einen pH-Modifikator, der Natriumbicarbonat ist; in einer Menge von etwa 200 mg bis etwa 600 mg;
gegebenenfalls einen Füllstoff, ausgewählt aus der Gruppe bestehend aus einem Zuckeralkohol, mikrokristalliner Cellulose, Methylcellulose, Hydroxypropylcellulose, Hydroxypropylmethylcellulose, Stärke, Dicalciumphosphat und einer Mischung davon; in einer Menge von etwa 0 mg bis etwa 60 mg; und
gegebenenfalls ein Fließregulierungsmittel, ausgewählt aus der Gruppe bestehend aus Silikonöl, Siliziumdioxid, Talkum, Magnesiumcarbonat und einer Mischung davon; in einer Menge von etwa 0 mg bis etwa 5 mg.

12. Zusammensetzung nach Anspruch 11, wobei:
kein Füllstoff vorhanden ist; und
das Fließregulierungsmittel Silikonöl ist; in einer Menge von etwa 0,1 mg bis etwa 5 mg.

13. Zusammensetzung nach einem der Ansprüche 9 bis 12, wobei die SDD eine mittlere Partikelgröße von etwa 5 µm bis etwa 113 µm Durchmesser aufweist.

14. Zusammensetzung nach Anspruch 11, wobei die Zusammensetzung umfasst:
eine SDD von etwa 30 Gew.-% von 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, 0,5-Ca-Hydrat; in einer Menge von etwa 1 mg, bezogen auf die freie Säure;
einen pH-Modifikator, der Natriumbicarbonat ist; in einer Menge von etwa 200 mg;
einen Füllstoff, der MCC PH-102 ist; in einer Menge von etwa 40 mg bis etwa 50 mg; und
ein Fließregulierungsmittel, das Silikonöl oder Siliciumdioxid ist; in einer Menge von etwa 1 mg bis etwa 5 mg;
wobei das Gesamtgewicht der Zusammensetzung etwa 250 mg beträgt.

15. Zusammensetzung nach Anspruch 11, wobei die Zusammensetzung umfasst:
eine SDD von etwa 30 Gew.-% von 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, 0,5-Ca-Hydrat; in einer Menge von etwa 6 mg, bezogen auf die freie Säure; und etwa 70 Gew.-% PVP-VA in einer Menge von etwa 14 mg;
einen pH-Modifikator, der Natriumbicarbonat ist; in einer Menge von etwa 600 mg; und
ein Fließregulierungsmittel, das Silikonöl ist; in einer Menge von etwa 1 mg bis etwa 10 mg.

16. Zusammensetzung nach Anspruch 11, wobei die Zusammensetzung umfasst:
eine SDD von etwa 30 Gew.-% von 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, 0,5-Ca-Hydrat; in einer Menge von etwa 12 mg, bezogen auf die freie Säure; und etwa 70 Gew.-% PVP-VA in einer Menge von etwa 28 mg;
einen pH-Modifikator, der Natriumbicarbonat ist; in einer Menge von etwa 600 mg; und
ein Fließregulierungsmittel, das Silikonöl ist; in einer Menge von etwa 1 mg bis etwa 10 mg.

17. Zusammensetzung nach Anspruch 11, wobei die Zusammensetzung umfasst:
eine SDD von etwa 30 Gew.-% von 3-[(1S,2S)-1-[5-[(4S)-2,2-Dimethyloxan-4-yl]-2-[(4S)-2-(4-fluor-3,5-dimethylphenyl)-3-[3-(4-fluor-1-methylindazol-5-yl)-2-oxoimidazol-1-yl]-4-methyl-6,7-dihydro-4H-pyrazolo[4,3-c]pyridin-5-carbonyl]indol-1-yl]-2-methylcyclopropyl]-4H-1,2,4-oxadiazol-5-on, 0,5-Ca-Hydrat; in einer Menge von etwa 36 mg, bezogen auf die freie Säure; und etwa 70 Gew.-% PVP-VA in einer Menge von etwa 83 mg;
einen pH-Modifikator, der Natriumbicarbonat ist; in einer Menge von etwa 600 mg; und
ein Fließregulierungsmittel, das Silikonöl ist; in einer Menge von etwa 1 mg bis etwa 10 mg.

18. Zusammensetzung nach einem der Ansprüche 14 bis 17, wobei die SDD eine mittlere Partikelgröße von etwa 5 µm bis etwa 113 µm Durchmesser aufweist.

19. Zusammensetzung nach einem Anspruch 18, wobei die SDD eine mittlere Partikelgröße von etwa 40 µm bis etwa 65 µm Durchmesser aufweist.

20. Zusammensetzung nach einem von Anspruch 19, wobei die Zusammensetzung in einer Kapselhülle vorliegt.

21. Zusammensetzung nach einem der Ansprüche 20, wobei die Kapselhülle eine HPMC-Kapselhülle ist.

22. Pharmazeutische Zusammensetzung nach einem der Ansprüche 1 bis 21 zur Verwendung in der Therapie.

23. Zusammensetzung zur Verwendung nach Anspruch 22, wobei die Therapie die Behandlung von Typ-2-Diabetes ist.

24. Zusammensetzung zur Verwendung nach Anspruch 22, wobei die Therapie die Gewichtskontrolle ist.

## Revendications

1. Composition de capsule orale solide comprenant une dispersion séchée par atomisation (SDD) de 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, ou d'un sel pharmaceutiquement acceptable de celle-ci ; et
un modificateur de pH choisi dans le groupe constitué de carbonate de calcium, carbonate de magnésium, bicarbonate de sodium, carbonate de sodium, hydroxyde de magnésium, hydroxyde de calcium, oxyde de magnésium, et un mélange de ceux-ci.

2. Composition selon la revendication 1, dans laquelle le modificateur de pH est choisi dans le groupe constitué de carbonate de calcium, carbonate de calcium anhydre, bicarbonate de sodium, bicarbonate de sodium anhydre, carbonate de sodium, carbonate de sodium anhydre, hydroxyde de magnésium et hydroxyde de magnésium anhydre.

3. Composition selon la revendication 1 ou la revendication 2, dans laquelle le modificateur de pH est du bicarbonate de sodium.

4. Composition selon la revendication 3, dans laquelle le modificateur de pH est du bicarbonate de sodium anhydre.

5. Composition selon l'une quelconque des revendications 1 à 4, dans laquelle la composition comprend
de la 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one ; et
du bicarbonate de sodium anhydre.

6. Composition selon l'une quelconque des revendications 1 à 4, dans laquelle la 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, ou un sel pharmaceutiquement acceptable de celle-ci, est
la 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, sel hémicalcique hydraté.

7. Composition selon la revendication 6, dans laquelle la 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, ou un sel pharmaceutiquement acceptable de celle-ci, est
la 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, sel hémicalcique hydraté en une quantité comprise d'environ 1,2 à environ 46 mg, sur une base d'acide libre, par composition de capsule.

8. Composition selon l'une quelconque des revendications 1 à 4, dans laquelle la 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, ou un sel pharmaceutiquement acceptable de celle-ci, est en une quantité comprise d'environ 1 mg à environ 45 mg, sur une base d'acide libre, par capsule.

9. Composition comprenant :
une dispersion séchée par atomisation (SDD) d'environ 30 % en poids à environ 35 % en poids de 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, ou d'un sel pharmaceutiquement acceptable de celle-ci ; en une quantité comprise d'environ 0,7 mg à environ 50 mg, sur une base d'acide libre ; et le reste de la SDD est composé de PVP-VA ; et
un modificateur de pH choisi dans le groupe constitué de carbonate de calcium, carbonate de magnésium, bicarbonate de sodium, carbonate de sodium, hydroxyde de magnésium, hydroxyde de calcium, oxyde de magnésium, et un mélange de ceux-ci.

10. Composition selon la revendication 9, dans laquelle la composition comprend :
la SDD d'environ 30 % en poids de 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, ou d'un sel pharmaceutiquement acceptable de celle-ci ; en une quantité comprise d'environ 0,7 mg à environ 45 mg, sur une base d'acide libre ;
un modificateur de pH qui est du bicarbonate de sodium ; en une quantité d'environ 150 mg à environ 650 mg ;
facultativement, une charge choisie dans le groupe constitué d'un alcool de sucre, de cellulose microcristalline, de méthylcellulose, d'hydroxypropylcellulose, d'hydroxypropylméthylcellulose, d'amidon, de phosphate dicalcique, et d'un mélange de ceux-ci ; en une quantité d'environ 0 mg à environ 200 mg ; et
facultativement, un agent glissant choisi dans le groupe constitué d'huile de silicone, dioxyde de silicium, talc, carbonate de magnésium, et un mélange de ceux-ci ; en une quantité d'environ 0 mg à environ 5 mg.

11. Composition selon la revendication 10, dans laquelle la composition comprend :
une SDD d'environ 30 % en poids de 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, sel hémicalcique hydraté ; en une quantité comprise d'environ 1 mg à environ 36 mg, sur une base d'acide libre ; et le reste de la SDD est composé de PVP-VA ;
un modificateur de pH qui est du bicarbonate de sodium ; en une quantité d'environ 200 mg à environ 600 mg ;
facultativement, une charge choisie dans le groupe constitué d'un alcool de sucre, de cellulose microcristalline, de méthylcellulose, d'hydroxypropylcellulose, d'hydroxypropylméthylcellulose, d'amidon, de phosphate dicalcique, et d'un mélange de ceux-ci ; en une quantité d'environ 0 mg à environ 60 mg ; et
facultativement, un agent glissant choisi dans le groupe constitué d'huile de silicone, dioxyde de silicium, talc, carbonate de magnésium, et un mélange de ceux-ci ; en une quantité d'environ 0 mg à environ 5 mg.

12. Composition selon la revendication 11, dans laquelle :
aucune charge n'est présente ; et
l'agent glissant est de l'huile de silicone ; en une quantité d'environ 0,1 mg à environ 5 mg.

13. Composition selon l'une quelconque des revendications 9 à 12, dans laquelle la SDD a une taille moyenne de particules d'environ 5 µm à environ 113 µm de diamètre.

14. Composition selon la revendication 11, dans laquelle la composition comprend :
une SDD d'environ 30 % en poids de 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, sel hémicalcique hydraté ; en une quantité d'environ 1 mg, sur une base d'acide libre ;
un modificateur de pH qui est du bicarbonate de sodium ; en une quantité d'environ 200 mg ;
une charge qui est de la MCC PH-102 ; en une quantité d'environ 40 mg à environ 50 mg ; et
un agent glissant qui est de l'huile de silicone ou du dioxyde de silicium ; en une quantité d'environ 1 mg à environ 5 mg ;
dans laquelle le poids total de la composition est d'environ 250 mg.

15. Composition selon la revendication 11, dans laquelle la composition comprend :
une SDD d'environ 30 % en poids de 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, sel hémicalcique hydraté ; en une quantité d'environ 6 mg, sur une base d'acide libre ; et environ 70 % en poids de PVP-VA en une quantité d'environ 14 mg ;
un modificateur de pH qui est du bicarbonate de sodium ; en une quantité d'environ 600 mg ; et
un agent glissant qui est de l'huile de silicone ; en une quantité d'environ 1 mg à environ 10 mg.

16. Composition selon la revendication 11, dans laquelle la composition comprend :
une SDD d'environ 30 % en poids de 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, sel hémicalcique hydraté ; en une quantité d'environ 12 mg, sur une base d'acide libre ; et environ 70 % en poids de PVP-VA en une quantité d'environ 28 mg ;
un modificateur de pH qui est du bicarbonate de sodium ; en une quantité d'environ 600 mg ; et
un agent glissant qui est de l'huile de silicone ; en une quantité d'environ 1 mg à environ 10 mg.

17. Composition selon la revendication 11, dans laquelle la composition comprend :
une SDD d'environ 30 % en poids de 3-[(1S,2S)-1-[5-[(4S)-2,2-diméthyloxan-4-yl]-2-[(4S)-2-(4-fluoro-3,5-diméthylphényl)-3-[3-(4-fluoro-1-méthylindazol-5-yl)-2-oxo-imidazol-1-yl]-4-méthyl-6,7-dihydro-4H-pyrazolo[4,3-c]pyridine-5-carbonyl]indol-1-yl]-2-méthylcyclopropyl]-4H-1,2,4-oxadiazol-5-one, sel hémicalcique hydraté ; en une quantité d'environ 36 mg, sur une base d'acide libre ; et environ 70 % en poids de PVP-VA en une quantité d'environ 83 mg ;
un modificateur de pH qui est du bicarbonate de sodium ; en une quantité d'environ 600 mg ; et
un agent glissant qui est de l'huile de silicone ; en une quantité d'environ 1 mg à environ 10 mg.

18. Composition selon l'une quelconque des revendications 14 à 17, dans laquelle la SDD a une taille moyenne de particules d'environ 5 µm à environ 113 µm de diamètre.

19. Composition selon la revendication 18, dans laquelle la SDD a une taille moyenne de particules d'environ 40 µm à environ 65 µm de diamètre.

20. Composition selon la revendication 19, dans laquelle la composition est dans une enveloppe de capsule.

21. Composition selon l'une quelconque des revendications 20, dans laquelle l'enveloppe de capsule est une enveloppe de capsule en HPMC.

22. Composition selon l'une quelconque des revendications 1 à 21 pour utilisation en thérapie.

23. Composition pour utilisation selon la revendication 22, dans laquelle la thérapie est le traitement de diabète de type 2.

24. Composition pour utilisation selon la revendication 22, dans laquelle la thérapie est une gestion du poids.
